Europäisches Patentamt

European Patent Office

Office européen des brevets

(19)

(11) Publication number: **0 107 518**
A2

(12) EUROPEAN PATENT APPLICATION

(21) Application number: 83306574.1

(22) Date of filing: 28.10.83

(51) Int. Cl.³: **A 61 H 7/00**

(30) Priority: 29.10.82 JP 190350/82

(43) Date of publication of application: 02.05.84
Bulletin 84/18

(84) Designated Contracting States: DE FR GB IT NL

(71) Applicant: **COSMO KOGYO KABUSHIKI KAISHA,**
2-1-25 Shimo-Shinzyo Nakahara-ku, Kawasaki-shi
Kanagawa-ken (JP)

(72) Inventor: **Makino, Yukio, 381 Kami-Aso Aso-ku,**
**Kawasaki-shi Kanagawa-ken (JP)**

(74) Representative: **Jennings, Nigel Robin et al, KILBURN &**
**STRODE 30 John Street, London WC1N 2DD (GB)**

(54) Skin treatment apparatus.

(57) A portable facial treatment apparatus includes a body (1)
and a rotary massage portion (9) connected to be rotated
by a motor (3). The body (1) contains infrared radiating
means (10) comprising a heater (11) and an infrared radiating
element (13) and steam generating means comprising water
absorbent material (15) situated adjacent the heater. A bellows
(17) is arranged to be reciprocated by the motor (3) and to blow
air past the absorbent material (15) to expel steam from the
body against the skin of the user.

1.

## SKIN TREATMENT APPARATUS

The present invention relates to apparatus for treating the skin, in particular the face, and is concerned with such apparatus of the type which includes a body and a massage portion connected to be actuated by a motor.

Conventional portable facial treatment apparatus as is used for promoting the recovery of skin from fatigue or removing secretions from the skin include a rotary massage portion for massaging the skin.

However, a simple massage of the skin from the exterior is not sufficient for an effective facial treatment, and it is found to be desirable to heat the skin from inside to speed up the circulation of blood, increase the metabolic rate and discharge unwanted matter, such as fats concentrated in the sebaceous glands and/or cosmetics, with sweat etc. thereby positively enhancing the refreshment of the skin.

The conventional facial treatment apparatus, however, applies a massage from the epidermis only and a full facial recovery effect is not obtained.

The present invention was developed with the above considerations in mind and has as its principal object the provision of an apparatus which produces a more effective beauty treatment and aims to provide a skin treatment apparatus for the face skin which can apply steam to the skin to warm it from the surface and make fat in the sebaceous glands soften and speed circulation of the blood, and can project infrared radiation onto the skin to warm it from deep inside the skin to further promote the blood circulation and to increase the metabolic rate. and can further apply rotational massage to the skin to stimulate the sebaceous glands or smooth out. wrinkles, or produce a synergistic effect of these various functions.

A further object of the invention is to provide a light weight small. portable facial treatment apparatus which can. perform all the functions referred to above and yet may be readily manipulated by hand.

According to the present invention apparatus for treating. the skin of the type. referred to above is characterised. in that the body contains infrared radiating means comprising a heater and an infrared radiating element and steam generating means comprising means which, in use, contains water adjacent. the heater. Thus the apparatus in accordance with the invention permits the skin to be massaged and also permits the simultaneous application of infrared radiation and steam to the skin. Such apparatus. is found. to be particularly useful for promoting the recovery of skin and rendering the skin fresh, highly limpid and both beautiful. and expressive.

The radiating surface of the infrared radiating element preferably comprises a layer of alumina and the massage portion is preferably connected to be rotated by the motor and is thus adapted to impart a rotary massage action to the skin.

In the preferred embodiment the steam generating means includes means such as a bellows arranged to blow air past the water containing means out of the body and thus to expel the steam from the body against the skin to be treated. The bellows may be connected to an actuator adapted to be reciprocated by the motor.

Further objects, features and advantages of the present invention will be apparent as from the following description of one specific embodiment which is given by way of example with reference to the accompanying drawings, in which:-

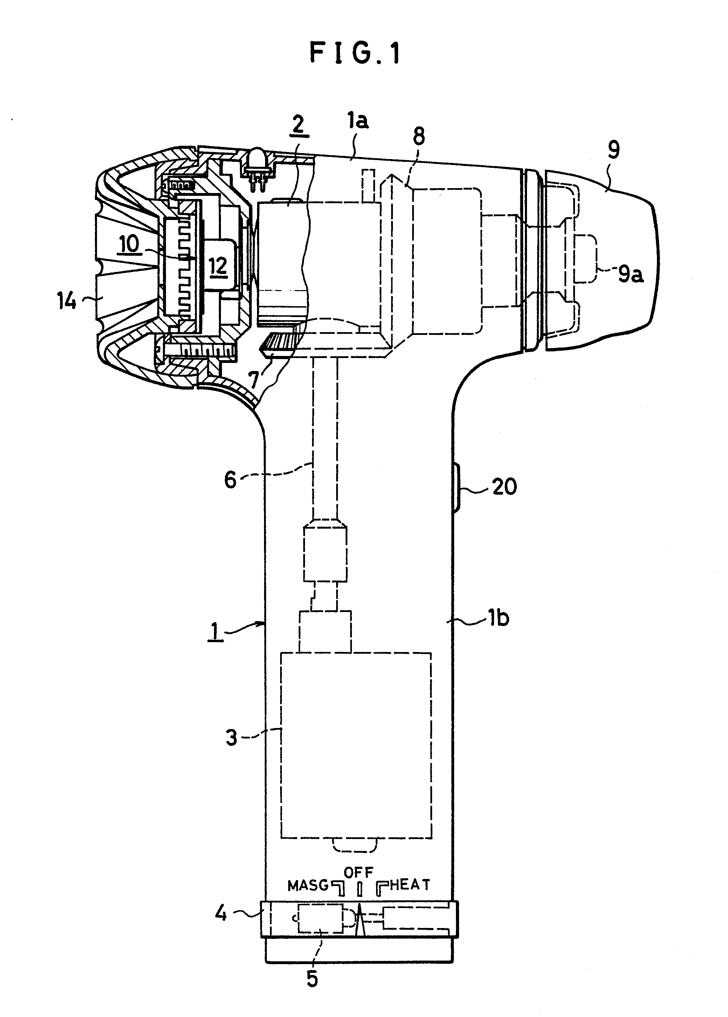

Figure 1 is a partly cut-away side view of a portable facial treatment apparatus in accordance with the present invention; and

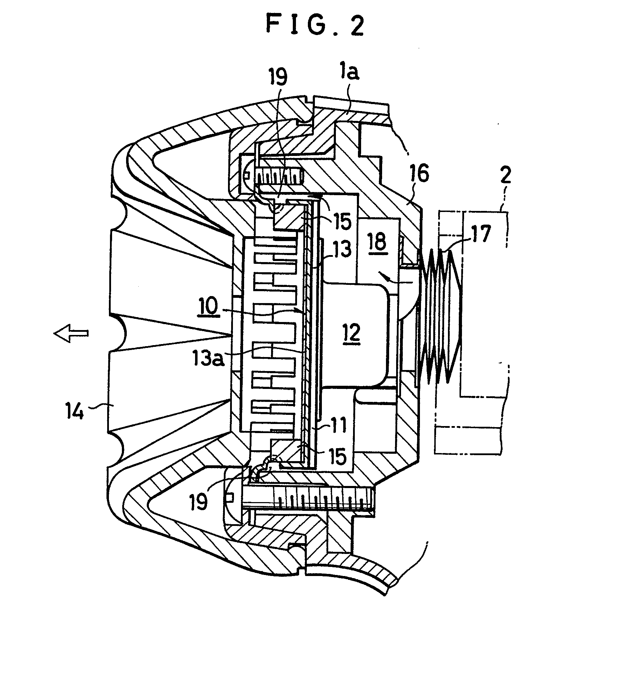

Figure 2 is an enlarged view of the cut-away portion of Figure 1.

The facial treatment apparatus includes an outer body 1 comprising a head 1a and a holder 1b. The head 1a contains an actuating mechanism 2 which is described in more detail below and which performs a massage function and the holder 1b contains a geared motor 3 connected to the mechanism 2 and to a rotary massage portion 9 of the housing through gears 7 and 8.

Adjacent the mechanism 2 is a far infrared ray radiating apparatus 10 including a circular disc state heater 11, a thermostat 12 which is connected directly to the rear of the heater and and a far infrared ray radiating portion 13 which is connected directly to the front of the heater and is substantially cup-shaped comprising a disc with a peripheral flange. Connected to the inner surface of the disc of the infrared radiating portion 13 is a radiation element 13a, e.g. of flame sprayed alumina. The radiation element 13a is, in this exemplary embodiment, 30 to 40µ thick and secured by adhesive to the radiating portion 13. When the temperature of the heater 11 reaches about 120°C, far infrared of a wave length of 8µ to 10µ, which is very easily absorbed by the human body, is radiated from the radiating element 13a in the direction of a beam cover or guide 14.

The far infrared ray radiating apparatus 10 is operated by heating the heater 11 by means of an external electric supply, e.g. the output voltage from an AC adapter, such as a 22V DC power source connected to a jack plug on the under side of the holder 1b. The temperature of the radiating element is maintained at 120°C ± 10°C by the thermostat 12.

Water containing or absorbing material 15 which is shaped to annular form and which, in use, holds water is situated around the periphery of the radiation element 13a between the infrared radiating portion 13 and cover 14. In use, the water in the water containing material 15 is evaporated by the heat from the heater 11, and the steam is projected from the beam cover 14 through the front surface of the head 1a, that is to say to the left as seen in the drawings.

5.

The mechanism 2 includes a piston which is arranged to be reciprocated by the motor 3. A bellows 17 is connected to the rear of a frame 16 to which far infrared radiation apparatus 10 is connected in such a manner that the bellows moves with the movement of the piston of the mechanism 2. The steam produced from the material 15 is expelled from the head 1a on each contraction of the bellows 17 by the air which is expelled from the bellows which flows out through a space 18 between the frame 16 and the infrared radiation apparatus 10 and through an appropriate number of air holes 19. The holes 19 are situated in the peripheral flange of the far infrared ray radiating portion 13 and positioned so that steam is entrained in the air expelled from the bellows and suitably projected against the skin of the face of the user when the beam cover is placed in contact with the skin. Air is sucked in to the bellows through a suction port situated beneath the rotary massage member 9 which is removable.

The infrared ray radiating element 13a is constructed to enhance far infrared radiation and to improve the infrared ray selection characteristics. The portion 13 is of cup shape and formed of aluminium material for good thermal conductivity, and the infrared radiation element 13a is prepared by plasma flame spraying (flame spraying temperature of about 20,000°C) of alumina which has superior far infrared radiation characteristics. To ensure that the peak value of the infrared radiation has a
wavelength of 8µ to 10µ, the thickness of the flame
sprayed alumina layer is 30µ to 40µ and the surface
of this layer is protected (top coated) with a resin
of the silicon acrylic series.  This feature serves
to improve the far infrared ray selection performance
and to increase the far infrared ray radiation
efficiency.

Reference numeral 4 designates a switch ring,
a slight sliding of which to either the left or
the right will cause a 3 position switch 5 to be
"ON".  Movement to the left produces only a
massage function by actuating a left hand feed
circuit to cause the motor to rotate the rotary
massage portion whilst movement to the right produces
a heating and massage function by actuating a right
hand feed circuit to rotate the rotary massage
portion and activate the heater 11, thereby producing
differing facial treatments.  It will be appreciated
that when the heat/massage circuit is activated
far infrared radiation is produced and optionally
steam also (if water is added to the water retaining
material 15) which is blown against the skin of the
user if the latter is adjacent the guide 14.

On the other hand, the rotary massage portion 9
is operated by rotation of the switch ring 4 in
either direction and can be used for skin massage
by pressing it against the skin surface.  If the
rotary massage portion 9, which is a removable
attachment, is removed from the head 1a, the suction

port 9a in the head 1a can be used to remove secretions from the skin and the like. The direction of rotation of the motor 3 may be reversed by means of a slide switch 20.

The skin treatment apparatus of the instant invention is thus more utilitarian and flexible in comparison with known apparatus which could be used only for the massage of the surface of the skin. Far infrared radiation, which is easily absorbed by the human body, can penetrate deep into the skin and thus a skin fatigue recovery effect may be applied within the skin promoting the blood circulation, and increasing the metabolic rate. Thus contaminants and other undesired matter such as greases and cosmetics accumulated in the sebaceous glands, are discharged with sweat to the surface of the skin. The use of steam in conjunction with infrared radiation promotes the beneficial effects both on the surface and within the skin. The apparatus is small and thus readily portable and may be easily handled.

8.

## CLAIMS

1. Apparatus for treating the skin including a body (1) and a massage portion (9) connected to be actuated by a motor (3), characterised in that the body (1) contains infrared radiating means (10) comprising a heater (11) and an infrared radiating element (13) and steam generating means comprising means (15) adjacent the heater (11) which, in use, contains water.

2. Apparatus as claimed in Claim 1 characterised in that the radiating surface of the infrared radiating element (13) comprises a layer (13a) of alumina.

3. Apparatus as claimed in Claim 1 or Claim 2 characterised in that the massage portion (9) is connected to be rotated by the motor (3) and adapted to impart a rotary massage action to the skin.

4. Apparatus as claimed in any one of the preceding claims characterised in that the steam generating means includes means (17) arranged to blow air past the water containing means (15) out of the body (1).

5. Apparatus as claimed in Claim 4 characterised in that the means for blowing air comprises a bellows (17) connected to an actuating means (2) adapted to be reciprocated by the motor (3).

6. Apparatus as claimed in Claim 4 characterised in that the massage portion (9) is removably connected to the body (1) and an air inlet aperture (9a) communicating with the bellows (17) is formed in the body (1) beneath the massage portion.

7. Apparatus as claimed in any one of the preceding claims characterised by a switch (5) connected to permit selective actuation of the massage portion (9) and/or the heater (11).

8. Apparatus as claimed in any one of the preceding claims characterised by a thermostat (12) arranged to maintain the temperature of the heater at between 110 and 130$^{\circ}$C whereby the infrared radiation has a wavelength of between 8$\mu$ and 10$\mu$.

KILBURN & STRODE
CHARTERED PATENT AGENTS
AGENTS FOR THE APPLICANTS

# FIG.1

# FIG. 2